# EUROPÄISCHE PATENTSCHRIFT

(11) **EP 0 428 650 B1**
(45) Veröffentlichungstag und Bekanntmachung des Hinweises auf die Patenterteilung: **15.09.1993**
(21) Anmeldenummer: 90906885.0
(22) Anmeldetag: 10.05.1990
(51) Int. Cl.: B29C 67/14, A61F 2/28, A61F 2/30, A61F 2/32

(54) **VERFAHREN ZUR HERSTELLUNG VON IMPLANTATEN**
PROCESS FOR MANUFACTURING IMPLANTS
PROCEDE POUR LA FABRICATION D'IMPLANTS

(30) Priorität: 15.06.1989 DE 3919508; 14.02.1990 DE 4004474
(43) Veröffentlichungstag der Anmeldung: 29.05.1991
(73) Patentinhaber: MAN Ceramics GmbH, 94453 Deggendorf (DE)
(72) Erfinder: DIERL, Rudolf, D-8060 Dachau (DE)
(86) Internationale Anmeldenummer: DE9000333
(87) Internationale Veröffentlichungsnummer: WO9015708

(56) Entgegenhaltungen:
- EP-A- 0 017 010
- WO-A-87/04916
- FR-A- 260 375
- FR-A- 2 222 199
- LU-A- 55 263
- US-A- 2 571 717
- US-A- 3 530 212
- US-A- 4 445 957

## Beschreibung

Die Erfindung bezieht sich auf ein Verfahren zur Herstellung von Verbundbauteilen, bei dem ein vorgefertigter Kern gemeinsam mit längs seiner Oberfläche geführten Verstärkungsfasern durch eine flexible Form geführt wird, wobei die Verstärkungsfasern an die Oberfläche des Kernes-angelegt werden.

Derartige Verfahren sind zur Herstellung von Verbundprofilen bekannt, die einen leichten Stützkern und einen faserverstärkten Mantel aufweisen, wobei die Fasern in Längsrichtung der Profile gerichtet sind.

Aus der DE 34 13 601 A1 ist ein Verfahren dieser Art bekannt, bei dem ein vorgefertigter Stützkern mit konstantem Querschnitt gemeinsam mit längs seiner Oberfläche geführten Verstärkungssträngen in einen Formkanal eingeführt wird, wobei vor oder nach dem Formkanal die Fasern mit Kunstharz getränkt werden und anschließend eine Aushärtung im kontinuierlichen Durchlaufbetrieb im Formkanal erfolgt, wobei das Kunstharz und die Fasern mit der Oberfläche des Stützkernes verbunden werden. Dieses ist ein kostengünstiges Herstellungsverfahren von länglichen Verbundprofilen mit längs der Oberfläche orientierten Fasern.

In der EP 17 010, auf die sich der Oberbegrift des Anspruchs 1 stützt wird ein Formkanal mit einer flexiblen, konisch verlaufenden Düse beschrieben, die das Durchziehen eines Faserbündels mit in Abständen eingeschlossenen, rhomboidförmigen Einzelelementen erlaubt, die eine relativ kleine Durchmessererweiterung des Stranges bewirken.

Der Erfindung liegt die Aufgabe zugrunde, ein fertigungstechnisch einfaches Verfahren zur Herstellung von Implantaten mit einer Ummantelung aus längsgerichteten Fasern zu schaffen.

Die Aufgabe wird erfindungsgemäß durch die Merkmale des Anspruchs 1 gelöst.

Die elastische Membran bildet eine dehnbare und kontrahierbare Form, die sich automatisch an den jeweiligen Querschnitt des Implantats anpaßt. Damit ist ein Verfahren geschaffen, mit dem unter Anwendung von gerätetechnisch sehr einfachen Ausführungen Implantate aus Verbundmaterial unregelmäßiger Geometrie mit einer Ummantelung aus längsgerichteten Fasern hergestellt werden können.

Dieses Verfahren eignet sich außerdem wegen des kontinuierlich möglichen Verfahrensablaufes bestens für eine Sereienfertigung von Implataten

Die Einbringung eines Matrixmaterials kann je nach Anwendungsfall durch Imprägnieren der Verstärkungsfasern oder durch Aufbringen des-Matrixmaterials auf den vorgefertigten Kern erfolgen.

Als Matrixmaterial wird vorzugsweise ein Harz verwendet, das bei Raumtemperatur erstarrt. Damit wird insbesondere bei Bauteilen mit kömplizierten Formen verhindert, daß die Fasern verrutschen oder sich vom Kern abheben.

Die Aushärtung erfolgt vorzugsweise durch eine im Anschluß an die Membran angebrachte Heizvorrichtung. Es ist aber selbstverständlich auch möglich, den mit Längsfasern ummantelten Kern in eine getrennte Heizvorrichtung zu bringen.

Gemäß einer weiteren Ausgestaltung der Erfindung wird das Implantat in einer Heizpressvorrichtung nachbehandelt, wobei das Implantat in seine Endform gebracht wird, während gleichzeitig das Matrixmaterial aushärtet.

Das erfindungsgemäße Verfahren eignet sich besonders zur Herstellung von schaftförmigen Implantatteilen, wie z. B. für Gelenkimplantate.

Die Erfindung wird anhand von in der Zeichnung schematisch dargestellten Ausführungsbeispielen näher beschrieben.

Es zeigen:
- Fig. 1A: Vorrichtungsteile, die in einem ersten Abschnitt des erfindungsgemäßen Verfahrens bei dessen Durchführung verwendet werden,
- Fig. 1B: Vorrichtungsteile, die in einem weiteren Abschnitt des erfindungsgemäßen Verfahrens bei dessen Durchführung verwendet werden,
- Fig. 1C: einen Längsschnitt durch ein mit dem erfindungsgemäßen Verfahren herstellbares stabförmiges Implantat nach dessen Ablängung vom Faserstrang,
- Fig. 1D: ein Beispiel für eine Nachbehandlung des in Fig. 1C gezeigten Implantats und
- Fig. 2 und 3: je ein bei der Durchführung des Verfahrens verwendetes Vorrichtungsteil.

Das erfindungsgemäße Verfahren wird nachfolgend an Hand der Herstellung eines Schaftes einer Hüftgelenk-Endoprothese beschrieben, der einen variierenden querschnitt hat. Ein solches Implantat kann, in Längsrichtung gesehen, gerade oder gekrümmt sein.

Bei der erfindungsgemäßen Herstellung eines solchen Implantats wird ein vorgefertiger Kern 1 mit einer Ummantelung 2 versehen, die aus Verstärkungsfasern 3 besteht, die der Länge nach um den Kern 1 verteilt werden. Die Zahl und der Querschnitt der einzelnen Verstärkungsfasern 3 richtet sich nach dem jeweiligen Anwendungsfall und den zu beherrschenden Beanspruchungen des Implantats.

Der Kern 1 kann im Prinzip aus irgendeinem Material hergestellt sein. Auch-dessen Ausgestaltung richtet sich nach dem jeweiligen Anwendungsfall.

Bei einem Kern aus einem aushärtbaren Material wird der vorgefertigte Kern 1 in der Regel in ausgehärteter Form dem Prozeß der Ummantelung mit den Verstärkungsfasern 3 unterzogen. Bei Bedarf ist es aber möglich, einen nicht ganz ausgehärteten Kern 1 zu verwenden, z. B. dann, wenn die Endkontur des Implantats nachträglich durch Wärme/Druck-Behandlung eingebracht werden soll. Dieses trägt gleichzeitig zur besseren Klebeverbindung Kern-Fasern bei.

Der Kern hat nach seiner Fertigungstellung eine Form, die der Kontur nach weitgehend jener der späteren Endform des Implantats entspricht, jedoch mit um die dicke der Ummantelung 2 reduzierten Außen- und Querschnittsabmessungen.

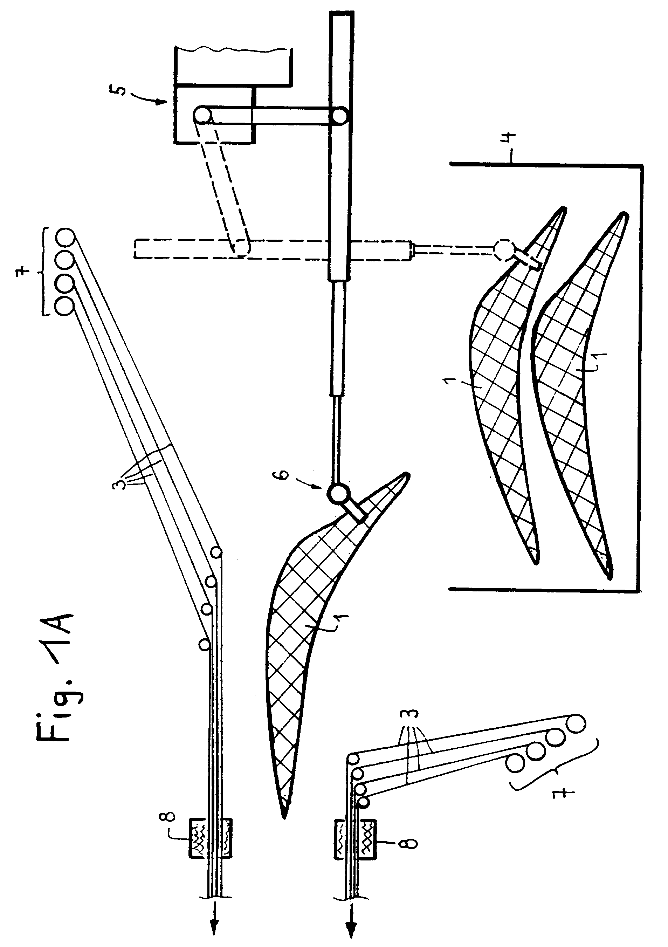

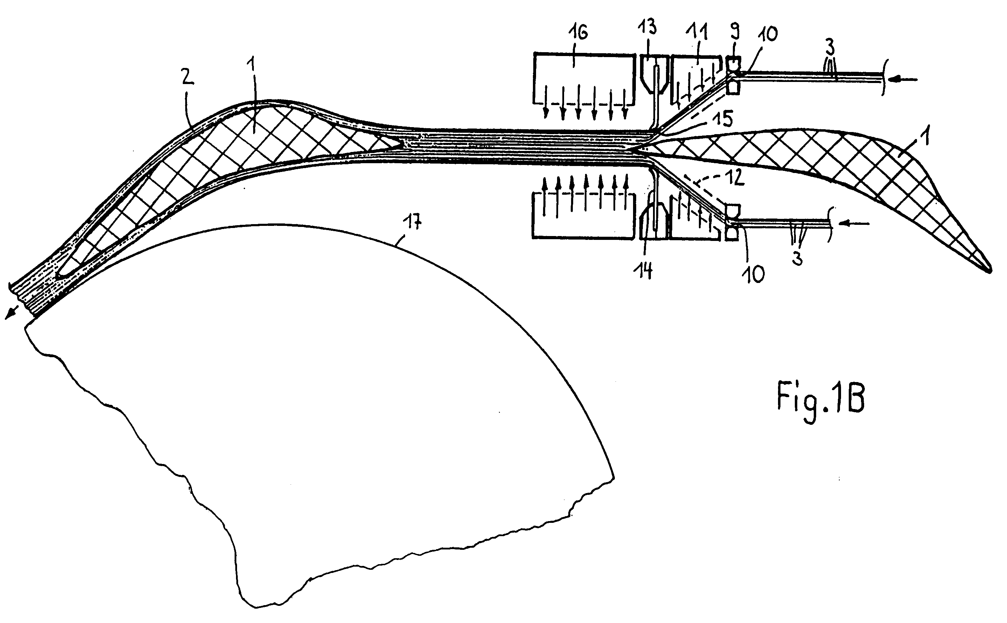

Die Verstärkungsfasern 3 sind in einem Magazin 7 in Endlosform auf Rollen aufgewickelt. Gemäß Fig. 1A ist den Verstärkungsfasern 3 eine Matrixmaterial enthaltende Tränkvorrichtung 8 zugeordnet, durch die die Fasern 3 geführt werden. Zusammen mit den Fasern 3 wird der Kern 1, wie in Fig. 1B näher gezeigt, durch die Öffnung 15 einer elastischen Membran 14 geführt, derart, daß die Fasern 3 in Längsrichtung des Bauteiles 1 auf dessen Oberfläche aufgelegt werden. Nach dem Durchziehen eines ersten Kernes 1 durch die Membran 14 wird, ohne die Fasern abzulängen, ein weiterer Kern 1 eingeführt.

Dieser Vorgang kann für eine Serienfertigung mechanisiert werden, indem, wie Fig. 1A gezeigt, die vorgefertigten Kerne in einem Magazin 4 bevorratet werden, woraus sie z. B. mittels eines automatisch angetriebenen Greifers 6 eines Roboters 5 zur Ummantelungsvorrichtung geführt werden.

Der Ummantelungsvorgang als solcher erfolgt mit einem gerätetechnisch sehr einfachen Aufwand, bei dem, je nach Notwendigkeit, zusätzliche Einrichtungen, wie Heiz- 11 und Kühleinrichtungen 16 ohne weiteres angebracht werden können.

In Fig. 1B ist vor Eintritt durch die Membran 14 eine Heizvorrichtung 11 vorgesehen, mit der das Matrixmaterial der Verstärkungsfaser 3 bis zur Zähflüssigkeit erwärmt werden kann. Falls notwendig, werden auf der inneren Seite der Faserführungen Abschirmungen 12 vorgesehen, um Wärmestrahlungen zum Kern 1 zu vermeiden. Die Membran 14 wird in einer einfachen Form mittels eines gestellfesten Ringes 13 gehalten. In Vorschubrichtung der Fasern 3 nach der Membran 14 ist eine Kühlvorrichtung 16 gezeigt.

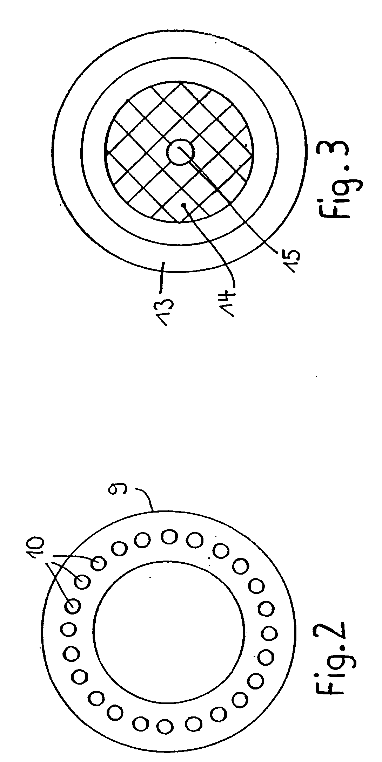

Die Verstärkungsfasern 3 werden grundsätzlich während des Ummantelns des Kernes 1 alle gleichzeitig und einzeln mit einer bestimmten Vorschubgeschwindigkeit von ihren Rollen aus dem Magazin 7 abgezogen und gegebenenfalls durch eine Tränkvorrichtung 8 geführt. Die Fasern sind dabei verteilt um den Kern 1 geführt. Dazu ist ein in Fig. 2 näher gezeigter Ring 9 mit entsprechend der Zahl der Fadenaugen 10 vorgesehen, die in regelmäßigen Abständen auf dem Ring 9 verteilt sind. Durch ein Fadenauge 10 können ein oder auch mehrere Fasern gleichzeitig geführt werden.

Die Membran 14 bildet beim Aufweiten eine trichterförmige Leitöffnung 15 und legt sich gleichmäßig an den Verstärkungsfasern 3 an, wobei überschüssiges Matrixmaterial abgestrichen und die Oberfläche der so entstandenen Randschicht geglättet wird, sofern die Verstärkungsfasern 3 vor deren Durchführung durch die Membran 14 mit Matrixmaterial imprägniert sind. Für die Matrix wird in der Regel ein der jeweiligen Anwendung entsprechendes Material verwendet. Um ein Verrutschen oder Abheben der Fasern am bzw. vom Kern weitestgehend zu verhindern, wird ein Heizsystem vorgeschlagen, das bei Temperaturen oberhalb der Raumtemperatur weich bis flüssig wird, bei Raumtemperatur bereits erstarrt. Damit lassen sich die Fasern kurz nach Austritt aus der Membran ausreichend am Kern fest verbinden, so daß die Faserposition am Kern erhalten bleibt.

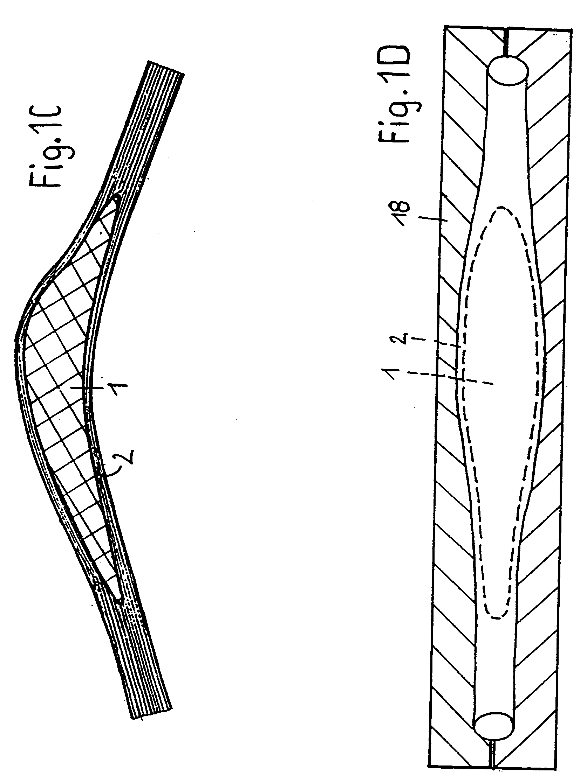

In Fig. 1C ist ein fertiggestellter Schaft 1, 2 einer Gelenkprothese gezeigt. Sofern erforderlich, kann der aus der Ummantelungsvorrichtung gezogene Schaft einer Nachbehandlung unterzogen werden.

Fig. 1D zeigt eine Nachbehandlung in einer Heißpressvorrichtung 18, in der der fertige Schaft 1, 2 unter Wärme- und Druckbeaufschlagung in seine äußere Endform gebracht und gleichzeitig das Matrixmaterial der Ummantelung 2, gegebenenfalls auch jenes der Kernes 1, ausgehärtet wird.

Fig. 3 zeigt schließlich eine Draufsicht auf die Membran 14, die im gestellfesten Ring 13 gehalten ist und eine Öffnung 15 zur Durchführung des zu ummantelnden Schaftes hat.

Die Ummantelung eines vorgefertigten Kernes 1 kann auch nur teilweise erfolgen, indem nicht durch alle Fadenaugen 10 Verstärkungsfasern 3 geführt werden. So können z. B. die Fadenaugen 10 einer Hälfte des Ringes 9 belegt werden. Es ist andererseits auch möglich, die Fadenaugen jeweils mit unterschiedlicher Zahl von Fasern zu bestücken, damit kann vom Querschnitt des fertigen Schaftes ausgehend, eine unterschiedliche Dicke der Ummantelung 2 erreicht werden.

## Patentansprüche

1. Verfahren zur Herstellung von Verbundbauteilen, bei dem ein vorgefertigter Kern (1) gemeinsam mit längs seiner Oberfläche geführten Verstärkungsfasern (3) durch eine flexible Form geführt wird, wobei die Verstärkungsfasern an die Oberfläche des Kernes angelegt werden, gekennzeichnet durch Anwendung des Verfahrens zur Herstellung von Knochenersatz-Implantaten (1, 2), wobei der vorgefertigte Kern (1) mit den Verstärkungsfasern (3) durch eine als elastische Membran (14) ausgebildete flexible Form durchgeführt wird.

2. Verfahren nach Anspruch 1, dadurch gekennzeichnet, daß der Kern (1) mit Matrixmaterial imprägnierten Verstärkungsfasern (3) durch die Membran (14) geführt wird.

3. Verfahren nach Anspruch 1 oder 2, dadurch gekennzeichnet, daß ein bei Raumtemperatur erstarrendes Matrixmaterial verwendet wird.

4. Verfahren nach einem der vorhergehenden Ansprüche, dadurch gekennzeichnet, daß das Implantat (1, 2) anschließend in einer Heißpressvorrichtung (18) nachbehandelt wird, wobei das Implantat in seine Endform gebracht und gleichzeitig das Matrixmaterial der Verstärkungsfasern (3) und gegebenenfalls auch jenes des Kernes (1) ausgehärtet werden.

5. Verfahren nach einem der vorhergehenden Ansprüche, dadurch gekennzeichnet, daß das Verfahren zur Herstellung eines Schaftes (1, 2) für ein Hüftgelenk-Implantat verwendet wird.

## Claims

1. A process for manufacturing composite components, wherein a pre-prepared core (1), together with reinforcing fibres (3) guided longitudinally over its surface, is guided through a flexible mould, the reinforcing fibres being applied to the surface of the core, **characterised by** use of the process to manufacture bone-replacement implants (1, 2), the pre-prepared core (1) with the reinforcing fibres (3) being guided through a flexible mould formed as a resilient membrane (14).

2. A process in accordance with claim 1, **characterised in that** the core (1), with reinforcing fibres impregnated with matrix material, is guided through the membrane (14).

3. A process in accordance with claim 1 or 2, **characterised in that** a matrix material is used which hardens at room temperature.

4. A process in accordance with one of the preceding claims, **characterised in that** the implant (1, 2) is subsequently treated in a hot-press device (18), the implant being brought into its final form, and the matrix material of the reinforcing fibres (3), and if appropriate, any matrix material of the core (1), being simultaneously hardened.

5. A process in accordance with one of the preceding claims, **characterised in that** the process is used for manufacturing a shaft (1, 2) for a hip joint implant.

## Revendications

1. Procédé pour la fabrication d'implant dans lequel un noyau (1) préfabriqué est passé à travers une forme flexible en commun avec des fibres de renforcement (3) guidées le long de sa surface, tandis que les fibres de renforcement sont appliquées sur cette surface du noyau, procédé caractérisé en ce qu'il est mis en oeuvre pour la réalisation d'implants de remplacement pour les os (1, 2), et en ce que le noyau préfabriqué (1) avec les fibres de renforcement (3) est passé à travers une forme flexible constituée par une membrane élastique (14).

2. Procédé selon la revendication 1, caractérisé en ce que le noyau (1) est passé à travers la membrane (14) avec des fibres de renforcement (3) imprégnées d'un matériau de matrice.

3. Procédé selon la revendication 1 ou la revendication 2, caractérisé en ce qu'on utilise un matériau de matrice se solidifiant à la température ambiante.

4. Procédé selon une des précédentes revendications, caractérisé en ce que l'implant est ensuite retraité dans un dispositif de pressage à chaud (18), en ce qu'il est amené à sa forme finale et qu'en même temps le matériau de matrice des fibres de renforcement (3), et éventuellement aussi celui du noyau (1) sont durcis.

5. Procédé selon une des précédentes revendications, caractérisé en ce qu'il est mis en oeuvre pour la réalisation d'un fût (1, 2) pour un implant de l'articulation de la hanche.
